# EUROPEAN PATENT APPLICATION

(11) **EP 2 617 424 A2**
(43) Date of publication of application: **24.07.2013**
(21) Application number: 13155909.8
(22) Date of filing: 23.08.2007
(51) Int. Cl.: A61K 31/52, A61K 31/522, A61P 17/02, A61P 25/00

(54) **Derivatives of uric and thiouric acid for oxidative stress-related diseases**

(30) Priority: 23.08.2006 US 839800 P
(62) Divisional of application: 07814379.9
(71) Applicant: Government of the United States of America, as represented by the Secretary, Department of Health and Human Services, Rockville, MD 20852 (US)
(72) Inventor: Greig, Nigel, H., Phoenix, MD Maryland 21311 (US); Mattson, Mark, P., Bel Air, MD Maryland 21015 (US); Haberman, Frank, Rishon Lezion, IL Illinois 75803 (US); Yu, Qian-sheng, Lutherville, MD Maryland 21093 (US); Arumugam, Thiruma, Baltimore, MD Maryland 21236 (US)
(74) Representative: Pfenning, Meinig & Partner GbR

(57) **Abstract**

Disclosed is a method of treating or preventing an oxidative stress-related disease in a mammal in need thereof comprising administering an effective amount of a compound of formula (I) to the mammal, wherein R¹-R⁴ and X¹-X³ are as described herein. Examples of the oxidative stress-related diseases are a neurological disorder, a cardiovascular disorder, and a wound.

## Description

### CROSS-REFERENCE TO RELATED APPLICATIONS

This patent application claims the benefit of U.S. Provisional Patent Application No. 60/839,800, filed August 23, 2006, which is incorporated by reference.

### BACKGROUND OF THE INVENTION

The risk of ischemic stroke, the second major cause of morbidity and mortality worldwide, can be reduced through diet and lifestyle modification, and the use of antihypertensive drugs (Ingall, T., J. Insur. Med., 36, 143-152 (2004)). However, the incidence of stroke remains high and there is a need for a treatment that can lessen the damage to brain cells and so improve functional outcome in those who suffer a stroke.

The mechanisms responsible for neuronal death caused by a stroke are believed to involve metabolic compromise, oxidative stress, over activation of glutamate receptors and cellular calcium overload (Zheng et al., Curr. Mol. Med., 3, 361-372 (2003)). Several different reactive oxygen species (ROS) are generated in neural cells following a stroke including superoxide anion radical, hydrogen peroxide, hydroxyl radical and nitric oxide (Lipton, P., Physiol. Rev., 79,1431-568 (1999) and Chan, P.H., J. Cereb. Blood Flow Metab., 21, 2-14 (2001)). ROS interact among themselves and with catalytic metals in ways that amplify damage to cellular membranes, proteins and nucleic acids resulting in cell damage and death. For example, hydrogen peroxide interacts with Fe²⁺ to generate hydroxyl radical, while superoxide anion radical interacts with nitric oxide to generate peroxynitrite. Hydroxyl radical and peroxynitrite are highly potent inducers of membrane lipid peroxidation an autocatalytic process involved in ischemic brain injury (Eliasson et al., J. Neurosci., 19, 5910-5918 (1999); Hall, Neurosurg. Clin. N. Am., 8, 195-206 (1997); Keynes et al., Curr. Mol. Med. 4, 179-191 (2004); and Selim et al., Ageing Res. Rev., 3, 345-353(2004)).

Thus, there remains a need for a method of treating oxidative stress caused by ROS. The invention provides such a method. This and other objects and advantages, as well as additional inventive features, will be apparent from the description of the invention provided herein.

### BRIEF SUMMARY OF THE INVENTION

The invention provides a method of treating or preventing an oxidative stress-related disease, including a neurological disorder, a cardiovascular disorder, or wound, in a mammal in need thereof comprising administering an effective amount of a compound of formula (I) to the mammal. Compounds of formula (I) are particularly useful in the treatment of oxidative stress-related diseases because of their improved water solubilities compared to uric acid.

### BRIEF DESCRIPTION OF THE SEVERAL VIEWS OF THE DRAWINGS

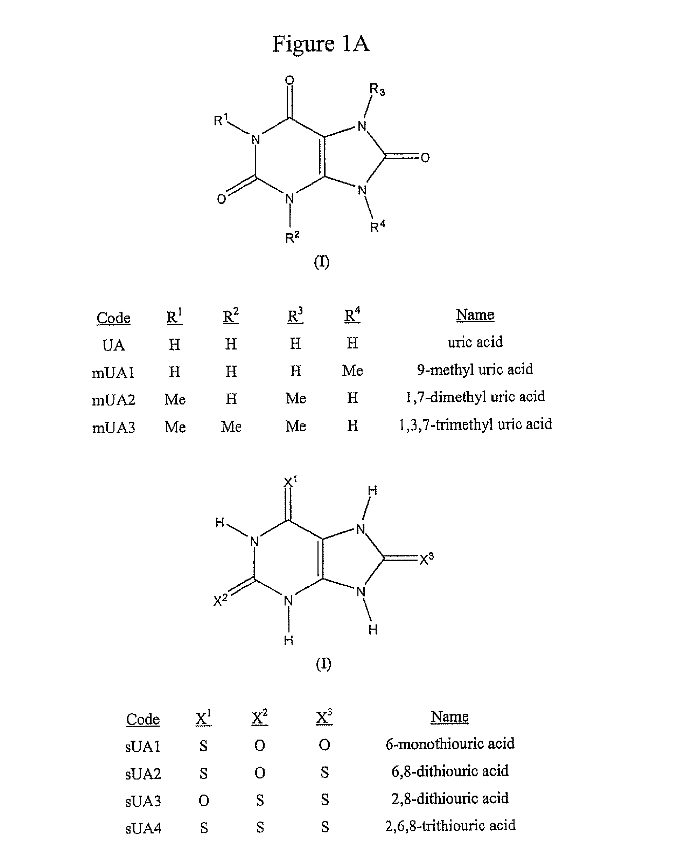

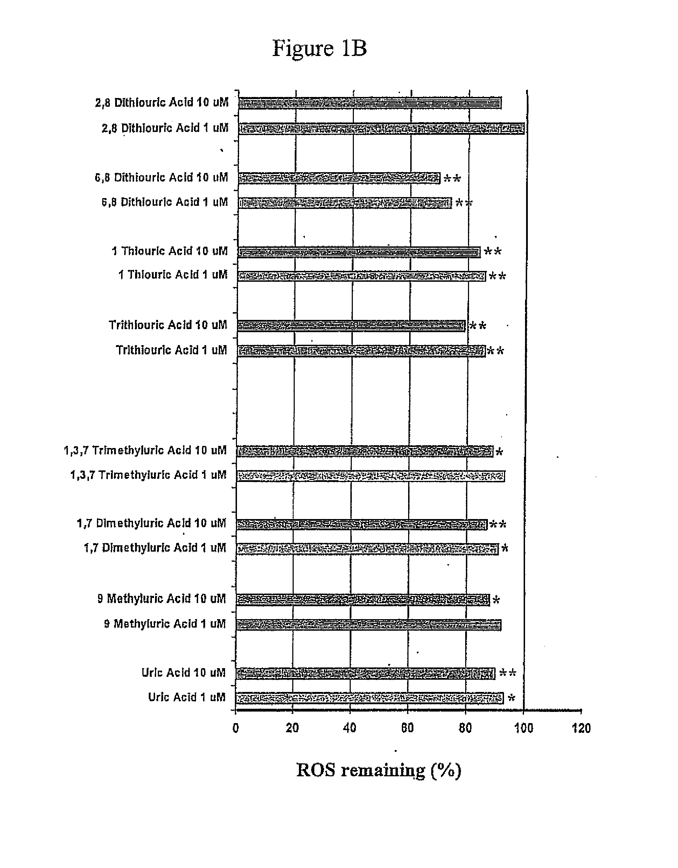

Figure 1A depicts structures of uric acid and exemplary compounds of formula (I). Figure 1B illustrates antioxidant activities of uric acid and compounds of formula (I) as quantified using a synaptosome assay in an embodiment of the invention. Levels of reactive oxygen species (ROS) are measured and values are expressed as a percentage of ROS present in Fe²⁺-treated control synaptosomes (mean and SEM, n=4 experiments). *p<0.05, **p<0.01 compared to the control value.

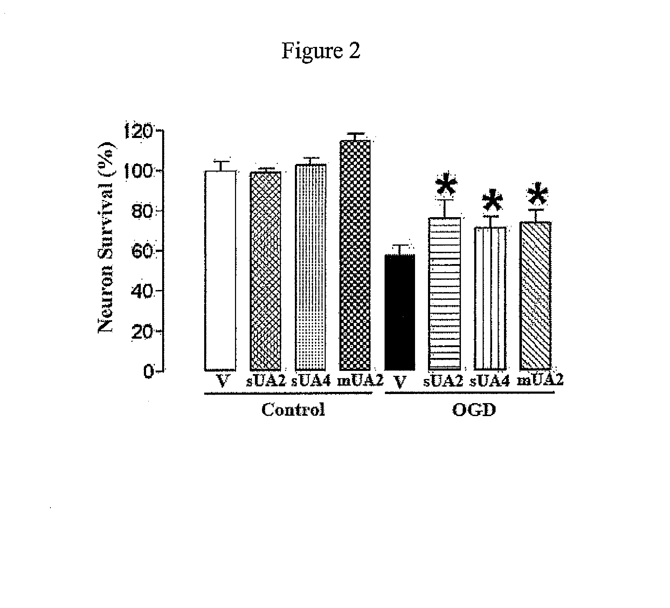

Figure 2 illustrates that compounds of formula (I) protect cultured hippocampal neurons against ischemia-like conditions in an embodiment of the invention. Neuronal survival is quantified and values are the mean and SEM (n=8-10 cultures). *p<0.05 compared to the vehicle/OGD cultures.

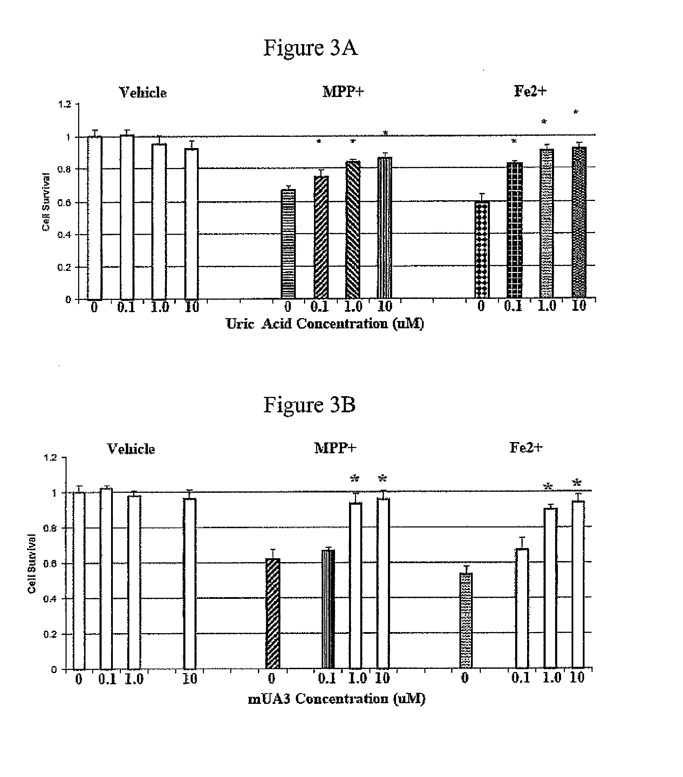

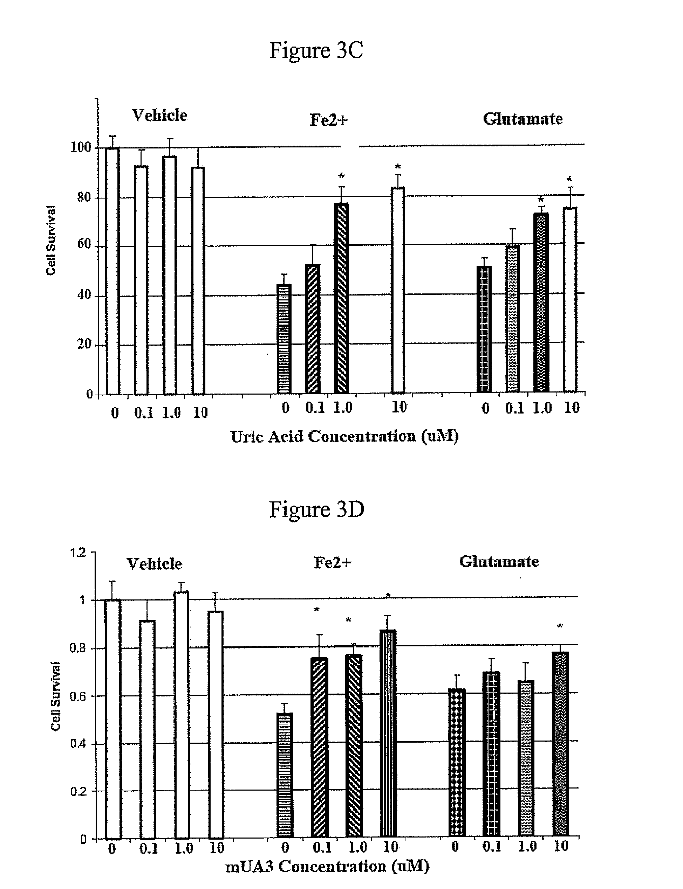

Figure 3 illustrates that uric acid and a compound of formula (I) protect cultured neural cells against oxidative and excitotoxic injury in an embodiment of the invention. Cultured PC12 cells are pretreated with uric acid (Figure 3A) or mUA3 (Figure 3B). Cultured hippocampal neurons are pretreated with uric acid (Figure 3C) or mUA3 (Figure 3D). Cell survival is quantified and values are the mean and SEM of 4-8 cultures. *p<0.05 compared to 0 uric acid or mUA3 concentration.

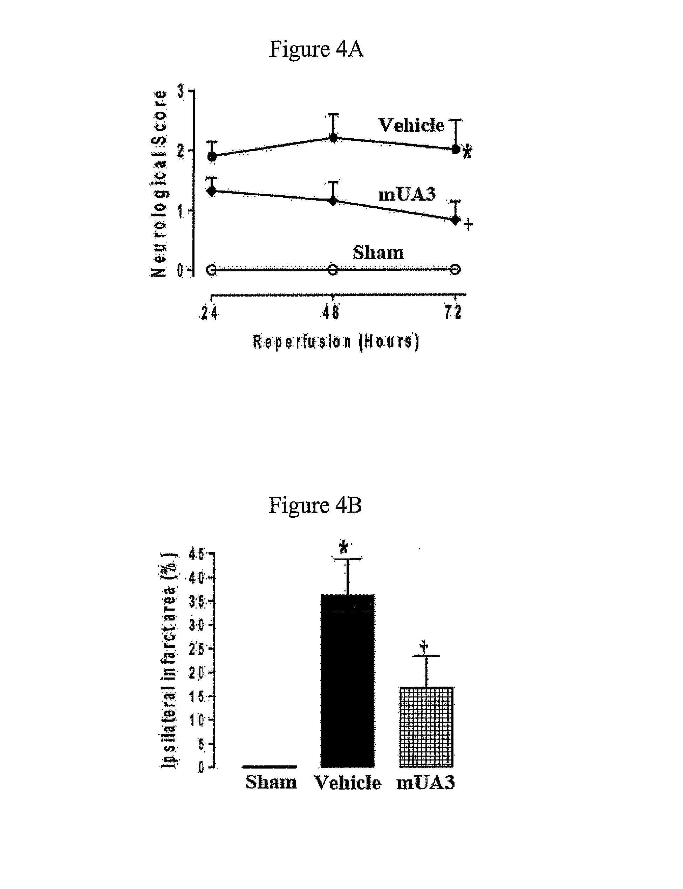

Figure 4 illustrates that a compound of formula (I) improves functional outcome (e.g., ambulation) and reduces brain damage in a mouse model of focal ischemic stroke in an embodiment of the invention. In Figure 4A neurological function is scored at 24, 48 and 72 hours post-stroke. In Figure 4B infarct volume is quantified at 72 hours post-stroke. Values are the mean and SEM (n= 8-12 mice). *p<0.001 compared to sham. +p<0.01 compared to vehicle.

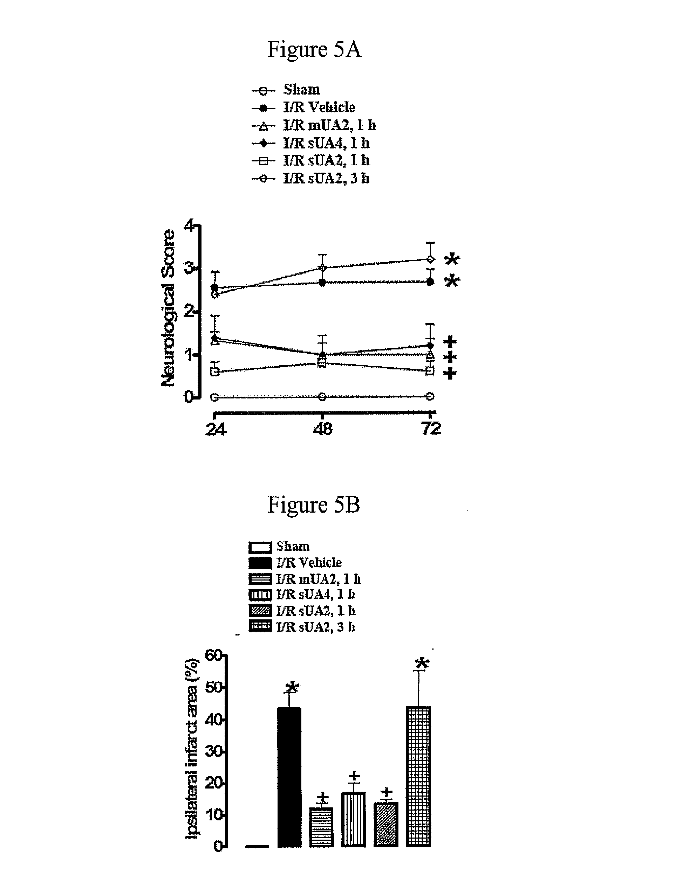

Figure 5 illustrates that compounds of formula (I) improve functional outcome and reduces brain damage in a mouse model of focal ischemic stroke in an embodiment of the invention. In Figure 5A, neurological function is scored at 24, 48 and 72 hours post-stroke. In Figure 5B, infarct volume is quantified at 72 hours post-stroke. Values are the mean and SEM (n= 8-12 mice). *p<0.001 compared to sham. +p<0.01 compared to vehicle.

### DETAILED DESCRIPTION OF THE INVENTION

Reactive oxygen species (ROS) have been implicated in a number of diseases or conditions, such as stroke, ischemia-reperfusion, arthritis, respiratory distress, cardiovascular diseases (e.g., myocardial infarction), carcinogenesis (e.g., cutaneous T-cell lymphomas, acute promyelocytic leukemia, squamous cell carcinomas of the skin, and basal cell carcinomas), and neurological diseases, including Parkinson's disease, dementia, Huntington's disease, Hodgkin's disease, and slow healing of wounds. The present invention provides compounds that protect cells from ROS and improve functional outcome.

Accordingly, the present invention provides a method of treating or preventing an oxidative stress-related disease in a mammal in need thereof comprising administering an effective amount of a compound of formula (I), or a composition thereof, to the mammal, wherein the compound of formula (I) is: 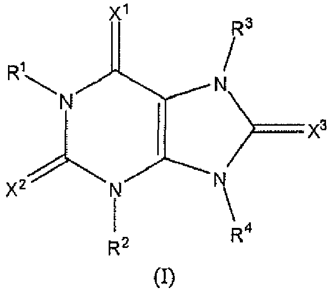 wherein
X¹, X², and X³ are the same or different and each is O or S; and
R¹, R², R³, and R⁴ are the same or different and each is hydrogen, C₁₋₆ alkyl, C₂₋₆ alkenyl, C₃₋₁₀ cycloalkyl, C₆₋₃₀ aryl, and C₆₋₃₀ aryl-C₁₋₆ alkyl, wherein the alkyl, alkenyl, cycloalkyl, aryl, or arylalkyl groups are optionally substituted with halo, amino, hydroxyl, acyl, nitro, carboxy, C₁₋₆ alkyl, carboxy-C₁₋₆ alkyl, hydroxy-C₁₋₆ alkyl, or C₁₋₆ alkoxy;
with the proviso that the compound of formula (I) is not uric acid.

The oxidative stress-related disease includes, for example, a neurological disorder, a cardiovascular disorder, and a wound. The cardiovascular disorder can be, for example, myocardial infarction, atherosclerosis, hyperlipidemia, diabetes mellitus, hypertension, and ischemic heart disease.

Within the context of treating or preventing oxidative stress-related diseases, the present invention provides a method of treating or preventing a neurological disorder in a mammal in need thereof comprising administering an effective amount of a compound of formula (I), or a composition thereof, to the mammal, wherein the compound of formula (I) is: 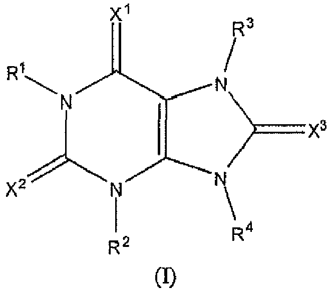 wherein
X¹, X², and X³ are the same or different and each is O or S; and
R¹, R², R³, and R⁴ are the same or different and each is hydrogen, C₁₋₆ alkyl, C₂₋₆ alkenyl, C₃₋₁₀ cycloalkyl, C₆₋₃₀ aryl, and C₆₋₃₀ aryl-C₁₋₆ alkyl,
wherein the alkyl, alkenyl, cycloalkyl, aryl, or arylalkyl groups are optionally substituted with halo, amino, hydroxyl, acyl, nitro, carboxy, C₁₋₆ alkyl, carboxy-C₁₋₆ alkyl, hydroxy-C₁₋₆ alkyl, or C₁₋₆ alkoxy;
with the proviso that the compound of formula (I) is not uric acid. The neurological disorder can be, for example, ischemic brain injury, trauma, epilepsy, arthritis, respiratory distress, Parkinson's disease, Huntington's disease, amyotrophic lateral sclerosis (ALS, also known as Lou Gehrig's disease), retinal degeneration (including retinitis pigmentosa, macular degeneration, and Usher syndrome), tauopathy (including Alzheimer's disease, progressive supranuclear palsy, corticobasal degeneration, and frontotemporal lobar degeneration (Pick's disease)), and dementia (including Alzheimer's disease, multi-infarct dementia (MID), dementia associated with Down's syndrome, and acquired immunodeficiency syndrome (AIDS) dementia).

When administered *in vivo* to a mammal, compounds of formula (I) accumulate in the brain at concentrations shown to be effective in protecting synaptosomes and cultured neurons against oxidative and ischemia-like insults. Thus, the compounds of formula (I) are particularly useful in the treatment of ischemic brain injury in a mammal. Ischemic brain injury according to the present invention can be due to short in oxygen supply to the brain, due to, for example, (i) short in blood supply to the brain, in cases of, for example, heart failure, delivery complications (fetus brain), and massive blood loss; (ii) short in blood oxygenation, due to, for example, short in atmosphere oxygen supply, CO poisoning, suffocation; or lung failure/obstruction; and (iii) hemorrhage in the brain or a region thereof, due to, for example, stroke or head trauma.

The present invention further provides a method of enhancing wound healing in a mammal in need thereof comprising administering an effective amount of a compound of formula (I), or a composition thereof, to the mammal, wherein the compound of formula (I) is: 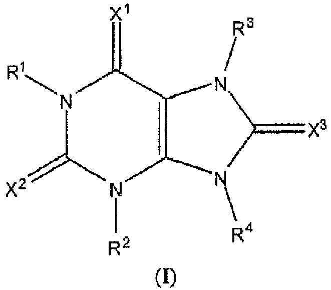 wherein
X¹, X², and X³ are the same or different and each is O or S; and
R¹, R², R³, and R⁴ are the same or different and each is hydrogen, C₁₋₆ alkyl, C₂₋₆ alkenyl, C₃₋₁₀ cycloalkyl, C₆₋₃₀ aryl, and C₆₋₃₀ aryl-C₁₋₆ alkyl,
wherein the alkyl, alkenyl, cycloalkyl, aryl, or arylalkyl groups are optionally substituted with halo, amino, hydroxyl, acyl, nitro, carboxy, C₁₋₆ alkyl, carboxy-C₁₋₆ alkyl, hydroxy-C₁₋₆ alkyl, or C₁₋₆ alkoxy;
with the proviso that the compound of formula (I) is not uric acid.

Enhancing wound healing includes, but is not limited to, acceleration of the wound healing process by decreasing oxidative stress upon administration of a compound of formula (I) compared to a control. Other mechanisms of wound healing are also contemplated. In an embodiment of this method, the compound of formula (I), or a composition thereof, is preferably administered topically to the wound. Wounds include cuts, abrasions, blemishes, ulcers, and burns.

In any of the embodiments of the invention, R¹, R², R³, and R⁴ are the same or different and each is hydrogen or C₁₋₆ alkyl, provided that at least one of R¹, R², R³, and R⁴ is C₁₋₆ alkyl when X¹, X², and X³ are all oxygen. Preferably, R¹, R², R³, and R⁴ are the same or different and each is hydrogen or methyl, provided that at least one of R¹, R², R³, and R⁴ is methyl when X¹, X², and X³ are all oxygen. In another embodiment of the invention, one, two, or all three of X¹, X², and X³ is sulfur. In any of the methods of the invention, preferably R¹, R², R³, and R⁴ are all hydrogen or all methyl. Specific embodiments of the compound of formula (I) are

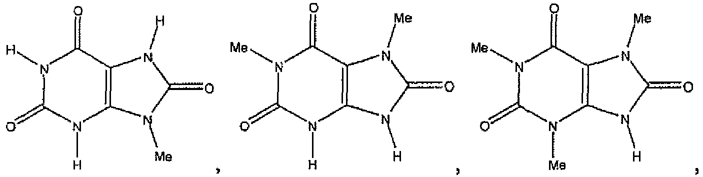

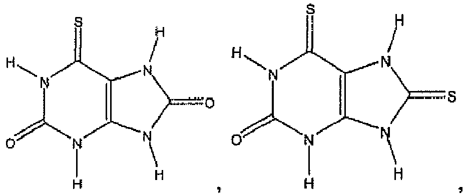

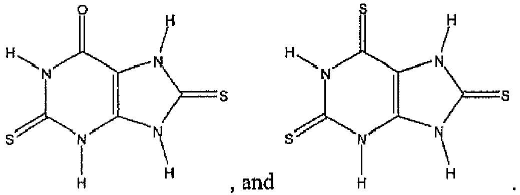

As regards the term "substituted" referring to the various groups in formula (I), any one of the groups that can be substituted (e.g., alkyl, alkenyl, cycloalkyl, aryl, or arylalkyl), wherever possible, generally can have 1 to 10 substituents (e.g., 1 to 8, 1 to 6, 1 to 4, 1 to 3 substituents) that are independently selected from the group consisting of halo, amino, hydroxyl, acyl, nitro, carboxy, C₁₋₆ alkyl, carboxy-C₁₋₆ alkyl, hydroxy-C₁₋₆ alkyl, or C₁₋₆ alkoxy.

The term "alkyl" refers to a straight or branched alkyl substituent, preferably having 1 to 3 carbon atoms. Examples of such substituents include methyl, ethyl, *n*-propyl, isopropyl, *n*-butyl, *sec*-butyl, isobutyl, *tert*-butyl, pentyl, isoamyl, hexyl, and the like.

The term "alkenyl," as used herein, means a linear or branched alkenyl substituent, preferably having 2 to 4 carbon atoms. Examples of such substituents include propenyl, isopropenyl, *n*-butenyl, *sec*-butenyl, isobutenyl, pentenyl, isopentenyl, hexenyl, and the like.

The term "cycloalkyl," as used herein, means a cyclic alkyl substituent having 3 to 10 carbon atoms, preferably 3 to 8 carbon atoms, preferably from 5 to 8 carbon atoms, more preferably from 5 to 6 carbon atoms. Examples of such substituents include cyclopropyl, cyclobutyl, cyclopentyl, cyclohexyl, and the like.

The term "aryl" refers to an unsubstituted or substituted aromatic carbocyclic substituent, as commonly understood in the art, and includes monocyclic and polycyclic aromatics such as, for example, phenyl, biphenyl, naphthyl, anthracenyl and the like. An aryl substituent generally contains from 6 to 30 carbon atoms, preferably from 6 to 18 carbon atoms, more preferably from 6 to 14 carbon atoms and further preferably from 6 to 10 carbon atoms. It is understood that the term aryl applies to cyclic substituents that are planar and comprise 4n+2 π electrons, according to Hückel's Rule.

The term "arylalkyl" refers to the group -R-Ar, in which R is an alkylene group having 1 to 6 carbon atoms, and Ar is an aryl group as described herein. An example of an arylalkyl is benzyl. The term "carboxyalkyl" refers to the group -RCOOH, in which R is an alkylene group having 1 to 6 carbon atoms. The term "hydroxyalkyl" refers to the group -ROH, in which R is an alkylene group having 1 to 6 carbon atoms.

The term "alkoxy" embraces linear or branched alkyl groups that are attached to an ether oxygen. The alkyl group is the same as described herein. Examples of such substituents include methoxy, ethoxy, propoxy, t-butoxy, and the like.

The term "halo" as used herein, means a substituent selected from Group VIIA, such as, for example, fluorine, bromine, chlorine, and iodine. Preferably, the halo is bromine or chlorine.

The compounds of formula (I) can be prepared by any suitable method, for example, by utilizing uric acid as a starting material. For example, one or more carbonyl groups on the compound of formula (I) can be converted to a thiocarbonyl by the use of Lawesson's reagent or by heating with phosphorus sulfide. The substituents R¹-R⁴ on the compound of formula (I) can be varied by any method known in the art (e.g., by nucleophilic substitution). For example, one or more of the nitrogens on the compound of formula (I) can be alkylated in the presence of an alkyl halide and base. Suitable reactions and reaction parameters are well known to those skilled in the art. See, e.g., Beaman et al., J. Org. Chem., 27, 986-990 (1962) and Maruyama et al., Nucleosides, Nucleotides, Nucleic Acids, 19, 1193-1203 (2000), which are incorporated herein by reference.

An advantage of the compounds employed in the embodiments of the invention is that they have increased water solubility. The water solubility of a compound of formula (I) generally is at least 0.2 mg/ml (e.g., at least 0.5 mg/ml, at least 0.8 mg/ml, at least 1 mg/ml, at least 1.5 mg/ml) as measured in water that has not been heated. Thus, the water solubility is from 0.2 to 5 mg/ml, preferably 1 to 5 mg/ml, and typically 2-3 mg/ml. Water solubility increases the ability, for example, to permeate cell membranes and bioavailability. Water solubility can be increased in hot water. Thus, the water solubility of a compound of formula (I) in heated water generally is at least 1 mg/ml (e.g., at least 1.5 mg/ml, at least 1.8 mg/ml, at least 2 mg/ml, at least 2.5 mg/ml).

The mammal can be any suitable subject with, or at risk of, an oxidative stress-related disease, including a neurological disorder or wound (e.g., a human, canine, feline, or simian). Preferably, the compound of formula (I) is administered before the oxidative stress-related disease has deleterious effects on the mammal or immediately upon determination of the deleterious effects of the oxidative stress-related disease (e.g., ischemic brain injury) and is administered continuously as needed.

An "effective amount" means an amount sufficient to show a meaningful benefit in an individual, e.g., promoting at least one aspect of inhibition of an oxidative stress-related disease, or treatment, healing, prevention, delay of onset, or amelioration of other relevant medical condition(s) associated with a particular oxidative stress-related disease. Effective amounts may vary depending upon the biological effect desired in the individual, condition to be treated, and/or the specific characteristics of the compound of formula (I), and the individual. In this respect, any suitable dose of the compound of formula (I) can be administered to the mammal, according to the type of oxidative stress-related disease to be treated. With respect to enhancing wound healing, the amount of compound of formula (I) necessary to treat wounded tissue (e.g., skin) is not fixed *per se,* and will be dependent upon the amount and type of any adjunct ingredients employed in the composition, the mamma's skin type, and the severity, extent, and nature of the wound treated.

Various general considerations taken into account in determining the "effective amount" are known to those of skill in the art and are described, e.g., in Gilman et al., eds., Goodman And Gilman's; The Pharmacological Bases of Therapeutics, 8th ed., Pergamon Press, 1990; and Remington's Pharmaceutical Sciences, 17th Ed., Mack Publishing Co., Easton, Pa., 1990, each of which is herein incorporated by reference. The dose of the compound of formula (I) desirably comprises about 0.1 mg per kilogram (kg) of the body weight of the mammal (mg/kg) to about 400 mg/kg (e.g., about 0.75 mg/kg, about 5 mg/kg, about 30 mg/kg, about 75 mg/kg, about 100 mg/kg, about 200 mg/kg, or about 300 mg/kg). In another embodiment, the dose of the compound of formula (I) comprises about 0.5 mg/kg to about 300 mg/kg (e.g., about 0.75 mg/kg, about 5 mg/kg, about 50 mg/kg, about 100 mg/kg, or about 200 mg/kg), about 10 mg/kg to about 200 mg/kg (e.g., about 25 mg/kg, about 75 mg/kg, or about 150 mg/kg), or about 50 mg/kg to about 100 mg/kg (e.g., about 60 mg/kg, about 70 mg/kg, or about 90 mg/kg).

The present inventive methods can also be practiced by administering a pharmaceutical composition comprising at least one compound of formula (I) and a pharmaceutically acceptable carrier. The composition can further comprise other active agents, such as adjuvants and other antioxidants. The antioxidants can be, for example, vitamins (e.g., vitamin C, vitamin E, vitamin A, and other retinoids), glutathione, superoxide dismutase-mimetic, a nitroxide, and beta carotene.

Any suitable pharmaceutically acceptable carrier can be used within the context of the invention, and such carriers are well known in the art. For example, the carrier comprises aqueous and non-aqueous solutions, a liquid that contains a buffer and a salt, sterile powders, granules, and tablets, a semipermeable matrix, a parenteral vehicle, or an intravenous vehicle. The choice of carrier will be determined, in part, by the particular site to which the pharmaceutical composition is to be administered and the particular method used to administer the pharmaceutical composition.

Suitable formulations include aqueous and non-aqueous solutions, isotonic sterile solutions, which can contain anti-oxidants, buffers, bacteriostats, and solutes that render the formulation isotonic with the blood or other bodily fluid of the intended recipient, and aqueous and non-aqueous sterile suspensions that can include suspending agents, solubilizers, thickening agents, stabilizers, and preservatives. In one embodiment, the pharmaceutically acceptable carrier is a liquid that contains a buffer and a salt. The formulation can be presented in unit-dose or multi-dose sealed containers, such as ampules and vials, and can be stored in a freeze-dried (lyophilized) condition requiring only the addition of the sterile liquid carrier, for example, water, immediately prior to use. Extemporaneous solutions and suspensions can be prepared from sterile powders, granules, and tablets.

Further carriers include sustained-release preparations, such as semipermeable matrices of solid hydrophobic polymers containing the active agent, which matrices are in the form of shaped articles (e.g., films, liposomes, or microparticles).

The pharmaceutical composition can include thickeners, diluents, buffers, preservatives, surface active agents, and the like.

The pharmaceutical composition comprising the compound of formula (I) can be formulated for any suitable route of administration, depending on whether local or systemic treatment is desired, and on the area to be treated. Desirably, the pharmaceutical composition is formulated for parenteral administration, such as intravenous, intraperitoneal, intraarterial, intrabuccal, subcutaneous, or intramuscular injection. Injectables can be prepared in conventional forms, either as liquid solutions or suspensions, solid forms suitable for suspension in liquid prior to injection, or as emulsions. Additionally, parental administration can involve the preparation of a slow-release or sustained-release system, such that a constant dosage is maintained. Preparations for parenteral administration include sterile aqueous or non-aqueous solutions, suspensions, and emulsions. Examples of non-aqueous solvents are propylene glycol, polyethylene glycol, vegetable oils, such as olive oil, and injectable organic esters, such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives also can be present such as, for example, antimicrobials, anti-oxidants, chelating agents, and inert gases and the like.

The pharmaceutical composition also can be administered orally. Oral compositions can be in the form of powders or granules, suspensions or solutions in water or non-aqueous media, capsules, sachets, or tablets. Thickeners, flavorings, diluents, emulsifiers, dispersing aids, or binders may be desirable.

Formulations for rectal administration can be presented as a suppository with a suitable base comprising, for example, cocoa butter or a salicylate. Formulations suitable for vaginal administration can be presented as pessaries, tampons, creams, gels, pastes, foams, or spray formulas. Similarly, the active ingredient can be combined with a lubricant as a coating on a condom. Indeed, preferably, the active ingredient is applied to any contraceptive device, including, but not limited to, a condom, a diaphragm, a cervical cap, a vaginal ring, and a sponge.

Suitable carriers and their formulations are further described in A.R. Gennaro, ed., Remington: The Science and Practice of Pharmacy (19th ed.), Mack Publishing Company, Easton, PA (1995).

The compound of formula (I) or a composition thereof can potentially be administered as a pharmaceutically acceptable acid-addition salt, formed by reaction with inorganic acids, such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid.

The compound or a pharmaceutical composition comprising a compound of formula (I) can be administered in or on a device that allows controlled or sustained release of the compound of formula (I), such as a sponge, biocompatible meshwork, mechanical reservoir, or mechanical implant. Implants (see, e.g., U.S. Patent 5,443,505), devices (see, e.g., U.S. Patent 4,863,457), such as an implantable device, e.g., a mechanical reservoir or an implant or a device comprised of a polymeric composition, are particularly useful for administration of the active agents. The pharmaceutical compositions of the inventive method also can be administered in the form of sustained-release formulations (see, e.g., U.S. Patent 5,378,475) comprising, for example, gel foam, hyaluronic acid, gelatin, chondroitin sulfate, a polyphosphoester, such as bis-2-hydroxyethyl-texephthalate (BHET), and/or a polylactic-glycolic acid. Of course, administration of the compound or pharmaceutical composition can be accomplished via any route that efficiently delivers the active agents to the target tissue.

The following examples further illustrate the invention but, of course, should not be construed as in any way limiting its scope.

### EXAMPLE 1

This example demonstrates a synthesis of a uric acid analog of formula (I) in an embodiment of the invention.

Commercially available derivatives are obtained from Sigma-Aldrich (St. Louis, MO). The methods used for the synthesis of methyl- and sulfur- derivatives of uric acid are similar to those described previously (Beaman et al., J. Org. Chem., 27, 986-990 (1962) and Maruyama et al., Nucleosides, Nucleotides, Nucleic Acids, 19, 1193-1203 (2000)) and structures are confirmed by ¹³C nuclear magnetic resonance (NMR) and ultra-violet (UV) spectral analyses. Uric acid analogs are assayed by high performance liquid chromatography (HPLC) and found to be of >97% purity. The solubilities of uric acid and compounds of formula (I) in cold water (mg/ml) are measured and set forth in Table 1.

**Table 1**

| **Compound** | **Water Solubility (mg/ml)** |
|---|---|
| uric acid (comparative) | 0.1 |
| mUA1 | 1 |
| mUA2 | 2 |
| mUA3 | 2 |
| sUA1 | 2 |
| sUA2 | 2 |
| sUA3 | 3 |
| sUA4 | 3 |

Thus, the water solubilities of the compounds of formula (I) are 10- to 30-fold greater than that of uric acid.

### EXAMPLE 2

This example demonstrates a method to determine the pharmacokinetics of compounds of formula (I) in an embodiment of the invention.

Mice are administered intravenously 10 mg/kg (5.95 mmols) of uric acid as a reference compound and equimolar doses of its analogs (sUA4 - 2, 6, 8 trithio uric acid, 12.9 mg/kg; mUA2 - 1,7 dimethyl uric acid, 11.7 mg/kg; sUA2 - 6,8 dithio uric acid, 11.9 mg/kg). Three month-old male C57BL/6 mice are injected with a compound and are euthanized 15, 30, 60 or 120 min later (1 mouse/time point/compound). Blood and brain tissues are rapidly removed, frozen on dry ice and kept at -80 °C before quantification of uric acid analogs. Plasma and cerebral cortical tissues are extracted in 10 volumes of 0.5 M perchloric acid and 1 mM ethylenediamine tetraacetic acid (EDTA). Levels of uric acid analogs in the extracts are quantified by HPLC methods employing a 25 cm RP C18 column, 25 mM KH₂PO₄ buffer (pH=4.6) with 1 ml/min flow rate and UV diode array detection.

Mice that are administered mUA2, sUA2 or sUA4 intravenously are euthanized at increasing time points (15, 30, 60 and 120 min) and levels of the compounds in plasma and brain tissue are measured. All three analogs (mUA2, sUA2 or sUA4) are detected in the brain at all time points examine. Their concentrations in the brain tissue (0.5 - 1 µM) are approximately 10% of the plasma concentrations at early time points (15-30 min), but remain elevated while plasma concentrations decrease progressively through the 120 min time point.

### EXAMPLE 3

This example demonstrates a method of synaptosome preparation and an assay to determine antioxidant activity in an embodiment of the invention.

Synaptosomes are prepared from the cerebral cortex of 2-3 month-old adult male Sprague-Dawley rats and are cryopreserved using methods described previously (Begley et al., Brain Res. Protoc., 3, 76- 82 (1998)). After thawing, cryopreserved synaptosomes are washed twice with Locke's buffer and plated in 24-well plates at a protein concentration of 250 µg per well. After a 30 min period to permit attachment of the synaptosomes to the substrate, synaptomoses are incubated for 1 h with compounds of formula (I) (1-10 µM), and then exposed for 4 h to freshly prepared 50 µM Fe²⁺ (FeSO₄). After an additional 30 min incubation in the presence of 10 µM dihydrorhodamine 123 (DHR), a probe for ROS which fluoresces when oxidized (Keller et al., J. Neurochem., 69, 273-284 (1997)), the synaptosomes are washed twice with Locke's buffer and levels of fluorescence are quantified using a fluorescence plate reader (488 nm excitation and 510 emission wavelengths). Fluorescence values for wells exposed to experimental treatments are expressed as a percentage of the mean value for untreated control wells. Levels of ROS in synaptosomes, measured using the fluorescent probe dihydrorhodamine, are significantly diminished by uric acid and all of the compounds of formula (I), except for sUA3 (Fig. 1B). Hippocampal neurons treated with sUA2, sUA4, and mUA2 are significantly more resistant to oxygen and glucose deprivation (OGD) induced death compared to neurons in control cultures (Fig. 2).

### EXAMPLE 4

This example demonstrates a method of preparing cell cultures and an assay to determine neuroprotection in an embodiment of the invention.

Methods for preparation and maintenance of cultures of PC12 cells and primary embryonic rat hippocampal neurons have been described previously (Furukawa et al., J. Neurosci., 17, 8178-8186 (1997) and Chan et al., J. Biol. Chem., 279, 28733-28743 (2004)). Cells are plated into polyethylenimine-coated plastic 96-well culture plates containing Neurobasal medium with B27 supplements. Experiments are performed in 7-8 day-old cultures (hippocampal neurons) or Dulbecco's modified eagle's medium (DMEM) with 10% fetal bovine serum (PC 12 cells). Hippocampal neurons and PC 12 cells are plated 7 days and 3 days prior to experiments, respectively. At the time of the experiments the cell density is approximately 100,000 (PC12 cells) and 50,000 (hippocampal neurons) cells per well. Immediately prior to experimental treatments, the culture medium is replaced with Locke's buffer. Cell viability is quantified by counting viable neurons in premarked microscope fields prior to and following experimental treatment and then calculating percent survival.

Cell death is induced by exposure of PC12 cells to either MPP⁺ or Fe²⁺. MPP⁺ is a neurotoxic metabolite of the Parkinsonian toxin MPTP which kills neurons by inhibiting mitochondrial complex I (Dauer et al., Neuron, 39, 889-909 (2003)), whereas Fe²⁺ induces hydroxyl radical production and membrane lipid peroxidation (Keller, J et al., J. Neurosci., 18, 687-697 (1998) and Keller et al., J. Neurochem., 69, 273-284 (1997)). Uric acid and mUA3 at concentrations of 0.1 to 10 µM protected PC12 cells against MPP⁺ and Fe²⁺ (Fig. 3A and 3B). Uric acid and mUA3 also protected primary hippocampal neurons against death induced by Fe²⁺ and glutamate excitotoxicity (Fig. 3C and 3D).

### EXAMPLE 5

This example demonstrates a mouse and focal cerebral ischemia-reperfusion model in an embodiment of the invention.

Three month-old male C57BL/6 mice are maintained on a 12 h light/12 h dark cycle with continuous access to food and water. The method for subjecting the mice to middle cerebral artery occlusion-reperfusion has been described previously (Arumugam et al., Am. J. Physiol. Heart Circ. Physiol., 287, H2555-2560 (2004)). Mice are anesthetized with isofluorane, a midline incision is made in the neck, and the left external carotid and pterygopalatine arteries are isolated and ligated with 5-0 silk thread. The internal carotid artery (ICA) is occluded at the peripheral site of the bifurcation of the internal carotid artery and the pterygopalatine artery with a small clip and the common carotid artery is ligated with 5-0 silk thread. The external carotid artery is cut, and a 6-0 nylon monofilament with a tip that is blunted (0.2-0.22 mm) with a coagulator is inserted into the external carotid artery. After the clip at the ICA is removed, the nylon thread is advanced into the middle cerebral artery until light resistance is felt. The nylon thread and the common carotid artery ligature are removed after 1 h of occlusion to initiate reperfusion. In the sham group, these arteries are visualized but not disturbed. Mice are administered either uric acid analogs of formula (I) or vehicle (dimethylsulfoxide) by infusion into the femoral vein (10 µl infused over a 2 min period) either 30 min prior to or 60 min after the onset of middle cerebral artery (MCA) occlusion. In a separate set of experiments anesthetized animals from all groups (4-6 mice per group) undergo cerebral blood flow (CBF) measurements using a laser Doppler perfusion monitor. All blood flow measurements are conducted with the mouse fixed in a plastic frame with the probe placed in the region of cerebral cortex perfused by the middle cerebral artery. These procedures are approved by the National Institute on Aging Animal Care and Use Committee.

When administered intravenously to mice at a dose of 20 mg/kg 60 min after the onset of middle cerebral artery occlusion, mUA3 significantly reduces brain damage and improves the functional outcome compared to vehicle-treated control mice (Fig. 4A and 4B). Infarct volumes are 40-60% lower in mice treated with mUA3 compared to vehicle-treated control mice. Another methyl analog (mUA2) and two sulfur analogs (sUA2 and sUA4) also significantly improve functional outcome and decrease brain damage when administered 1 h after ischemia onset (Fig. 5A and 5B). There are no significant differences in CBF between vehicle and mice treated with a compound of formula (I) before, during or after MCA occlusion (data not shown), suggesting that the neuroprotective effects of the analogs are not the result of an effect on the cerebral vasculature.

### EXAMPLE 6

This example demonstrates an evaluation of neurological deficits and brain injury in an embodiment of the invention.

The functional consequences of focal cerebral ischemia-reperfusion injury are evaluated by using a five-point neurological deficit score (0, no deficit; 1, failure to extend right paw; 2, circling to the right; 3, falling to the right; and 4, unable to walk spontaneously) and are assessed in a blinded fashion. At 72 h of reperfusion, the mice are killed with a lethal dose of isofluorane. The brains are immediately removed and placed into PBS (4°C) for 15 min, and 2-mm coronal sections are cut with a tissue cutter. The brain sections are stained with 2% 2,3,5-triphenyltetrazolium chloride in phosphate buffer at 37 °C for 15 min. The stained sections are photographed, and the digitized images are used for analysis. The borders of the infarct in each brain slice are outlined and the area quantified using a NIH image 6.1 software. To correct for brain swelling, the infarct area is determined by subtracting the area of undamaged tissue in the left hemisphere from that of the intact contralateral hemisphere. Infarct volume is calculated by integration of infarct areas for all slices of each brain.

### EXAMPLE 7

This example demonstrates an evaluation of wound healing with a compound of formula (I) in an embodiment of the invention.

A defined wound is created with a 4 mm Accuderm human punch biopsy kit (Accuderm, Ft Lauderdale, FL) in C57BL/6 mice. Specifically, the loose skin on the back of a mouse was brought together and, under anesthetic, a single punch was made through either side to create two wounds through the skin alone - without affecting underlying tissue. Immediately post-wounding, the wounds are either treated topically with normal saline (to serve as the non-treated control group) or with a compound of formula (I).

Each day the wounds are digitally photographed and wound areas are determined by computer integration of the photographs. Wound area at the time of wounding (day 0) is arbitrarily set to a relative value of 1 for all wounds; such that subsequent wound areas are converted to relative wound areas by dividing the wound area at day n by the wound area at day zero.

The application of a single dose of the compound of formula (I) (at the time of wounding, day zero) accelerates the time to full wound closure in the mouse model. On average, wounds treated with 6, 8-dithiouric acid (sUA2) closed in 8 days post wounding compared to greater than 13 days in the saline treated control.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.
Further Embodiments:
1. A method of treating or preventing an oxidative stress-related disease in a mammal in need thereof comprising administering an effective amount of a compound of formula (I) to the mammal, wherein the compound of formula (I) is: 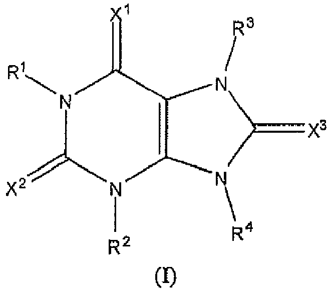 wherein
   X¹, X², and X³ are the same or different and each is O or S; and
   R¹, R², R³, and R⁴ are the same or different and each is hydrogen, C₁₋₆ alkyl, C₂₋₆ alkenyl, C₃₋₁₀ cycloalkyl, C₆₋₃₀ aryl, and C₆₋₃₀ aryl-C₁₋₆ alkyl
   wherein
   the alkyl, alkenyl, cycloalkyl, aryl, or arylalkyl groups are optionally substituted with halo, amino, hydroxyl, acyl, nitro, carboxy, C₁₋₆ alkyl, carboxy-C₁₋₆ alkyl, hydroxy-C₁₋₆ alkyl, or C₁₋₆ alkoxy;
   with the proviso that the compound of formula (I) is not uric acid.
2. The method of claim 1, wherein the oxidative stress-related disease is a neurological disorder.
3. The method of claim 2, wherein the neurological disorder is selected from the group consisting of ischemic brain injury, trauma, epilepsy, arthritis, respiratory distress, Parkinson's disease, Huntington's disease, amyotrophic lateral sclerosis, retinal degeneration, tauopathy, and dementia.
4. The method of claim 2 or 3, wherein the neurological disorder is selected from the group consisting of ischemic brain injury, trauma, epilepsy, arthritis, respiratory distress, Parkinson's disease, Huntington's disease, amyotrophic lateral sclerosis, and dementia.
5. The method of claim 3 or 4, wherein the dementia is associated with Alzheimer's disease, multi-infarct dementia (MID), Down's syndrome, or acquired immunodeficiency syndrome (AIDS).
6. The method of claim 3, wherein the neurological disorder is ischemic brain injury.
7. The method of claim 1, wherein the oxidative stress-related disease is a wound.
8. A method of treating or preventing a neurological disorder in a mammal in need thereof comprising administering an effective amount of a compound of formula (I) to the mammal, wherein the compound of formula (I) is: 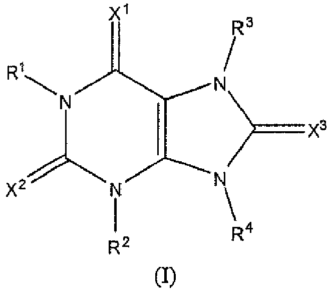 wherein
   X¹, X², and X³ are the same or different and each is O or S; and
   R¹, R², R³, and R⁴ are the same or different and each is hydrogen, C₁₋₆ alkyl, C₂₋₆ alkenyl, C₃₋₁₀ cycloalkyl, C₆₋₃₀ aryl, and C₆₋₃₀ aryl-C₁₋₆ alkyl
   wherein
   the alkyl, alkenyl, cycloalkyl, aryl, or arylalkyl groups are optionally substituted with halo, amino, hydroxyl, acyl, nitro, carboxy, C₁₋₆ alkyl, carboxy-C₁₋₆ alkyl, hydroxy-C₁₋₆ alkyl, or C₁₋₆ alkoxy;
   with the proviso that the compound of formula (I) is not uric acid.
9. A method of enhancing wound healing in a mammal in need thereof comprising administering an effective amount of a compound of formula (I), or a composition thereof, to the mammal, wherein the compound of formula (I) is: 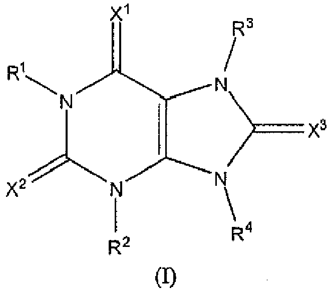 wherein
   X¹, X², and X³ are the same or different and each is O or S; and
   R¹, R², R³, and R⁴ are the same or different and each is hydrogen, C₁₋₆ alkyl, C₂₋₆ alkenyl, C₃₋₁₀ cycloalkyl, C₆₋₃₀ aryl, and C₆₋₃₀ aryl-C₁₋₆ alkyl
   wherein
   the alkyl, alkenyl, cycloalkyl, aryl, or arylalkyl groups are optionally substituted with halo, amino, hydroxyl, acyl, nitro, carboxy, C₁₋₆ alkyl, carboxy-C₁₋₆ alkyl, hydroxy-C₁₋₆ alkyl, or C₁₋₆ alkoxy,
   with the proviso that the compound of formula (I) is not uric acid.
10. The method of any of claims 1-9, wherein R¹, R², R³, and R⁴ are the same or different and each is hydrogen or C₁₋₆ alkyl, provided that at least one of R¹, R², R³, and R⁴ is C₁₋₆ alkyl when X¹, X², and X³ are all oxygen.
11. The method of any of claims 1-10, wherein R¹, R², R³, and R⁴ are the same or different and each is hydrogen or methyl, provided that at least one of R¹, R², R³, and R⁴ is methyl when X¹, X², and X³ are all oxygen.
12. The method of any of claims 1-11, wherein one, two, or all three of X¹, X², and X³ is sulfur.
13. The method of any of claims 1-12, wherein R¹, R², R³, and R⁴ are hydrogen.
14. The method of any of claims 1-12, wherein R¹, R², R³, and R⁴ are methyl.
15. The method of any of claims 1-11, wherein the compound of formula (I) is
   selected from the group consisting of 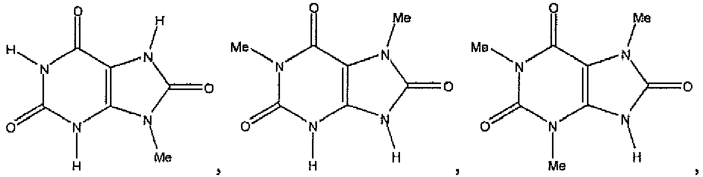 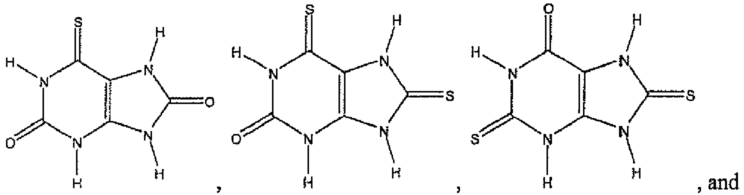 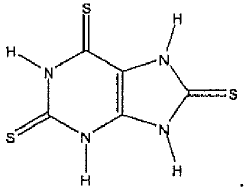
16. Use of a compound of formula (I) in the preparation of a medicament for the treatment or prevention of an oxidative stress-related disease, wherein the compound of formula (I) is: 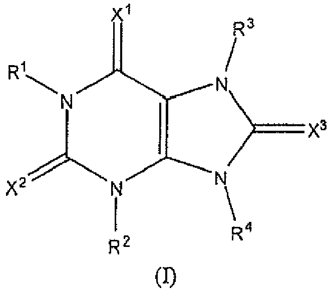
wherein
X¹, X², and X³ are the same or different and each is O or S; and
R¹, R², R³, and R⁴ are the same or different and each is hydrogen, C₁₋₆ alkyl, C₂₋₆ alkenyl, C₃₋₁₀ cycloalkyl, C₆₋₃₀ aryl, and C₆₋₃₀ aryl-C₁₋₆ alkyl
wherein
the alkyl, alkenyl, cycloalkyl, aryl, or arylalkyl groups are optionally substituted with halo, amino, hydroxyl, acyl, nitro, carboxy, C₁₋₆ alkyl, carboxy-C₁₋₆ alkyl, hydroxy-C₁₋₆ alkyl, or C₁₋₆ alkoxy;
with the proviso that the compound of formula (I) is not uric acid.

## Claims

1. A compound of formula (I) for use in treating a wound or enhancing wound healing in a mammal, 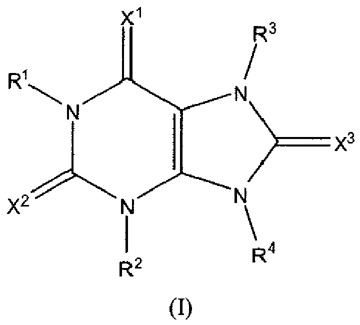 wherein
X¹, X², and X³ are the same or different and each is O or S; and
R¹, R², R³, and R⁴ are the same or different and each is hydrogen, C₁₋₆ alkyl, C₂₋₆ alkenyl, C₃₋₁₀ cycloalkyl, C₆₋₁₀ aryl, and C₆₋₃₀ aryl-C₁₋₆ alkyl; wherein the alkyl, alkenyl, cycloalkyl, aryl, or arylalkyl groups are optionally substituted with halo, amino, hydroxyl, acyl, nitro, carboxy, C₁₋₆ alkyl, carboxy-C₁₋₆ alkyl, hydroxy-C₁₋₆ alkyl, or C₁₋₆ alkoxy;
with the proviso that the compound of formula (I) is not uric acid.

2. A compound of formula (I) for use in treating or preventing an oxidative stress-related disease in a mammal, wherein the oxidative stress-related disease is a neurological disorder, arthritis, respiratory disease, or retinal degeneration, 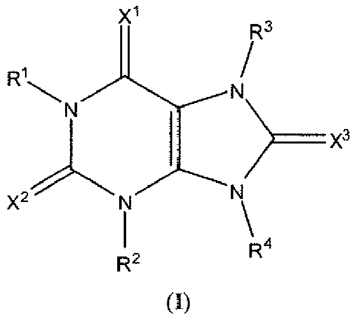 wherein
X¹, X², and X³ are the same or different and each is O or S; and
R¹, R², R³, and R⁴ are the same or different and each is hydrogen, C₁₋₆ alkyl, C₂₋₆ alkenyl, C₃₋₁₀ cycloalkyl, C₆₋₁₀ aryl, and C₆₋₃₀ aryl-C₁₋₆ alkyl; wherein the alkyl, alkenyl, cycloalkyl, aryl, or arylalkyl groups are optionally substituted with halo, amino, hydroxyl, acyl, nitro, carboxy, C₁₋₆ alkyl, carboxy-C₁₋₆ alkyl, hydroxy-C₁₋₆ alkyl, or C₁₋₆ alkoxy;
with the proviso that the compound of formula (I) is not uric acid.

3. The compound of claim 1 or 2, wherein R¹, R², R³, and R⁴ are the same or different and each is hydrogen or C₁₋₆ alkyl, provided that at least one of R¹, R², R³, and R⁴ is C₁₋₆ alkyl when X¹, X², and X³ are all oxygen.

4. The compound of any one of claims 1-3, wherein R¹, R², R³, and R⁴ are the same or different and each is hydrogen or methyl, provided that at least one of R¹, R², R³, and R⁴ is methyl when X¹, X², and X³ are all oxygen.

5. The compound of any one of claims 1-4, wherein one, two, or all three of X¹, X², and X³ are sulfur.

6. The compound of any one of claims 1-5, wherein R¹, R², R³, and R⁴ are hydrogen.

7. The compound of any one of claims 1-6, wherein R¹, R², R³, and R⁴ are methyl.

8. The compound of any one of claims 1-7, wherein the compound of formula (I) is selected from the group of consisting of
